# EUROPEAN PATENT APPLICATION

(11) **EP 0 528 282 A2**
(43) Date of publication of application: **24.02.1993**
(21) Application number: 92113427.6
(22) Date of filing: 06.08.1992
(51) Int. Cl.: A61F 13/15

(54) **Disposable absorbent article**

(30) Priority: 08.08.1991 US 742776
(71) Applicant: KIMBERLY-CLARK CORPORATION, Neenah, Wisconsin 54956-0349 (US)
(72) Inventor: Bruemmer, Mary Anne, Neenah, Wisconsin 54956 (US)
(74) Representative: Diehl, Hermann O. Th., Dr.

(57) **Abstract**

A disposable article (12) is provided that has a waistband (38) at one end that can be fastened about the waist of a wearer that is standing up. The portion of the article (12) hanging downwardly from the waistband (38) is then passed between the legs and attached at the back of the wearer. Stretchable leg cuffs (16) are provided at least at the crotch area (22) of the article (12) and extend laterally outwardly beyond the lateral sides of the article.

## Description

This invention pertains to a disposable absorbent article.

Currently, disposable absorbent articles find widespread use in infant, child, and adult incontinence care, and have generally replaced reusable cloth absorbent articles. A typical disposable absorbent article generally comprises a three-layered composite structure comprising a topsheet, a backsheet, and an absorbent between the topsheet and backsheet. Two examples of such absorbent articles are baby diapers and adult incontinence garments.

Although current diapers or other absorbent articles have been generally accepted by the public, these articles still have need of improvement in certain areas. Specifically, mothers have indicated a desire for a baby diaper that can be fitted to their baby while the baby is standing up. Generally, these babies that are able to stand will be of an age between about 18 months to about 36 months, and can be uncooperative in wanting to lie down to be changed. This problem can occur in other circumstances, such as in a restaurant or other building that does not have diaper changing facilities. In both of these instances, a diaper that could be changed while the baby is standing would provide greater convenience, less of a mess, and likely assist in the transition to toilet training.

Although three-dimensional training pant designs offer standing application, they require the removal of outer clothing in order to be removed and replaced with a clean pant.

Mothers are also concerned about their babies opening and removing their diapers before they are ready to begin the toilet training process. This problem naturally can lead to messy and embarrassing situations when the baby unfastens a soiled diaper. This occurs with conventional diapers because their fastening tapes are located on the front side of the diaper, and the baby can easily see and reach the fastening tapes in order to unfasten them and remove the diaper.

In order to solve this problem the invention provides a disposable article according to independent claim 1, 20, or 26. Further advantageous features, aspects, and details are evident from the dependent claims, the description and the drawings. The claims are intended to be understood as a first non-limiting attempt of defining the invention in general terms.

The invention provides a disposable diaper enabling standing application with childproof fastening.

The invention also provides a two-dimensional disposable absorbent article that can be fited onto a wearer standing up.

In one form of the invention there is provided a disposable absorbent article comprising a front section, a back section, a crotch section, and a pair of opposed lateral sides. An absorbent medium is disposed on the crotch section, and a stretchable leg cuff is disposed on each lateral side in such a way that the stretchable leg cuff extends laterally, outwardly beyond each lateral side. A waist-securing member is disposed with either the front section or the back section, and can be attached or secured about the waist of the wearer.

The above-mentioned and other features of the present invention, and the manner of obtaining them, will become more apparent and the invention itself will be better understood by reference to the following detailed description of the invention taken in conjunction with the accompanying drawings, wherein:
Fig. 1 is a front elevational view of a conventional diaper on the torso of a wearer;
Fig. 2 is a side elevational view of Fig. 1;
Fig. 2A is a perspective view of the diaper in Fig. 1 in its relaxed condition prior to being fitted onto a wearer;
Fig. 3 is a front elevational view of a diaper according to an embodiment of the present invention on a wearer;
Fig. 4 is a side elevational view of Fig. 3;
Fig. 5 is a front elevational view of a diaper according to an embodiment of the the present invention, the diaper being fitted onto the wearer;
Fig. 6 is a top plan view of a modification of the Fig. 14 embodiment and without a topsheet;
Fig. 7 is a sectional view of the modification of Fig. 6 with a topsheet taken along line 7-7 and viewed in the direction of the arrows;
Fig. 8 illustrates a variation to the view in Fig. 7;
Fig. 9 illustrates another variation to the view in Fig. 7;
Fig. 10 is a top plan view of another modification of the Fig. 14 embodiment;
Fig. 11 is a top plan view of another modification of the Fig. 14 embodiment and without a topsheet;
Fig. 12 is a top plan view of another modification of the Fig. 14 embodiment;
Fig. 13 is a side elevational view of the modification in Fig. 12 fitted onto a wearer; and
Fig. 14 is a top plan view of a preferred embodiment of the present invention.

### Definitions

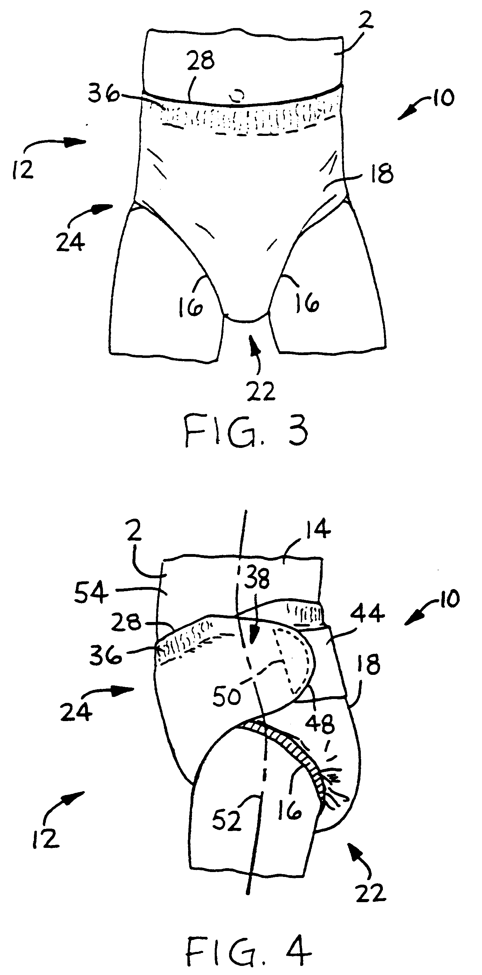

Within the context of the present disclosure, each term below will include the following meaning:
(a) "Disposed", "disposed on", "disposed with", "disposed at", and any variations thereof are intended to mean that one element can be integral with another element, or that one element can be a separate structure joined to or placed with or near another element.
(b) "Non-gathered" describes the effect of the stretchable leg cuffs of the present invention on the material or structure on which they are disposed, and is in contrast to the effect of leg elastics that are attached in the conventionally stretched condition to a diaper structure which, upon relaxing the stretched, attached leg elastics, is gathered. In the present invention, the stretchable leg cuffs are attached so that they do not gather the layer to which they are attached, as do the leg elastics in a conventional diaper.
(c) "Cohesive" describes a material or composition that is self-sticking in that it only adheres to itself.
(d) "Back", "back side", and "back portion" with reference to the human anatomy is defined by reference to Fig. 4. Fig. 4 illustrates a centerline or plane 52 passing through the center of the illustrated torso to divide it in half. The "back" or "back side" or "back portion" of the wearer will include that portion from the centerline on one side of the wearer and around the back to a similar point on the other side of the wearer.
(e) "Front", "front side", and "front portion" include the front part of the torso in Fig. 4 complementary to the above-defined "back" or "back side" or "back portion".
(f) "Stretch-bonded laminate" (SBL) means at least a two-layered composite in which one layer is a gatherable layer and the other layer a stretchable layer. The layers are joined together when the stretchable layer is in a stretched condition so that upon relaxing the layers, the gatherable layer is gathered. The stretchable layer can be a film of stretchable material or a plurality of strands of a stretchable material.
(g) "Two-dimensional" refers to a garment that can be opened and laid in a flat condition. These garments are not manufactured with closed leg openings and require a fastening device to attach about the wearer.
(h) "Three-dimensional" garments are similar to shorts or pants in that they have leg openings that are continuous, i.e., are bounded by the material of which they are made.

These definitions are not intended to be limiting, and these terms may be defined with additional language in the remaining portion of the specification.

### Detailed Description

The two-dimensional disposable absorbent article of the present invention will be described in terms of a baby diaper designed to fit a baby in the age range between about 18 months to about 36 months. Babies in this age range generally will weigh between about 11.3 kg to about 15.9 kg (about 25 lbs. to about 35 lbs).

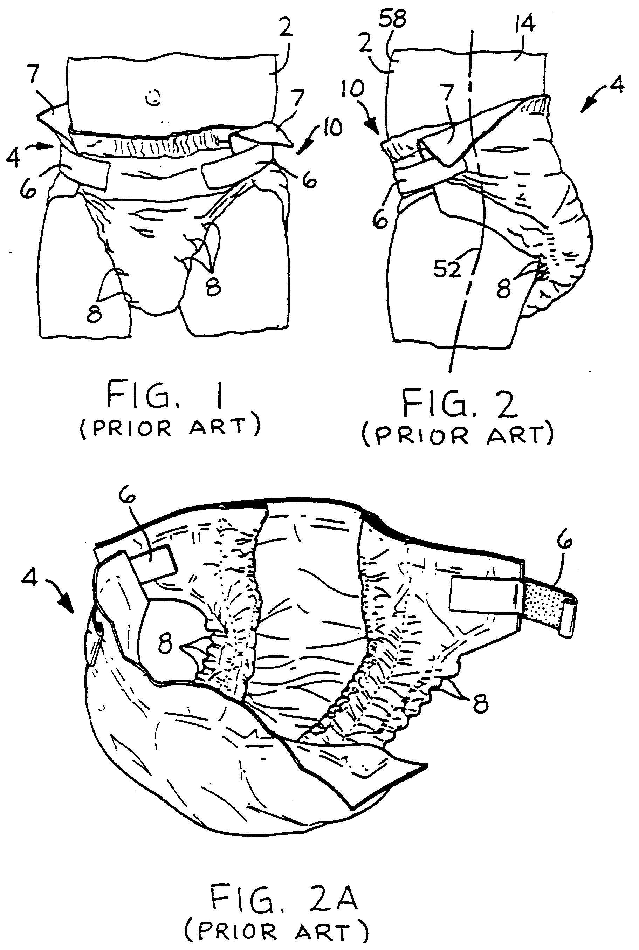

Referring to Figs. 1, 2, and 2A, torso 2 has conventional diaper 4 fitted thereon by means of fastening tapes 6. Because of the generally narrow width, i.e., the vertical dimension as viewed in Figs. 1 and 2, of fastening tape 6 in relation to the width, or vertical dimension, of that portion of the diaper ears that they join together, the top of the diaper along waistline 10 tends to fold over upon itself, thereby creating folds 7. The crotch area of diaper 4 between the legs is also in a gathered or rumpled state. The gathering or rumpling exists due to the method of attaching leg elastics to a conventional diaper. For a given weight range and size range of the baby to be fitted by a single diaper, a conventional diaper is made to have a length sufficient to fit the largest baby in that size and weight range. Then, in order to fit the smallest baby in that size and weight range and still maintain some degree of leakage control at the leg openings, a conventional diaper at the crotch section is gathered by attaching leg elastics in a stretched condition to the liquid impermeable backsheet and/or the liquid permeable topsheet. Upon relaxing this diaper after attaching the leg elastics in this manner, the leg elastics will gather the topsheet and/or backsheet. This gathering shortens a conventional diaper in length so that it can fit the smallest size baby. As the baby size increases from the smallest to the largest for a given weight and size range, the diaper is increasingly stretched to fit the baby. The result of this is that conventional diaper 4 may not provide a neat appearance and, for the smaller babies in the specific size and weight range, does not provide a trim fit at the crotch area that would assist in leakage control.

In comparison, a disposable absorbent diaper 12 of the present invention, as illustrated in Figs. 3 and 4, provides a neater appearance and a more trim fit. As viewed from the front in Fig. 3, it can be seen that the fastening devices for disposable diaper 12 are not visible because they attach at back portion 14 of the wearer. Diaper 12 is not only neater and more trim fitting at waistline 10, but also at the leg or crotch area because it is designed to fit the smallest baby in that specific size and weight range. Thus, there is no additional or excess material, such as a backsheet and/or a topsheet, at the crotch area that requires gathering by leg elastics. To fit the larger babies in this size and weight range, diaper 12 is provided with a pair of stretchable leg cuffs 16 that easily stretch to fit the baby.

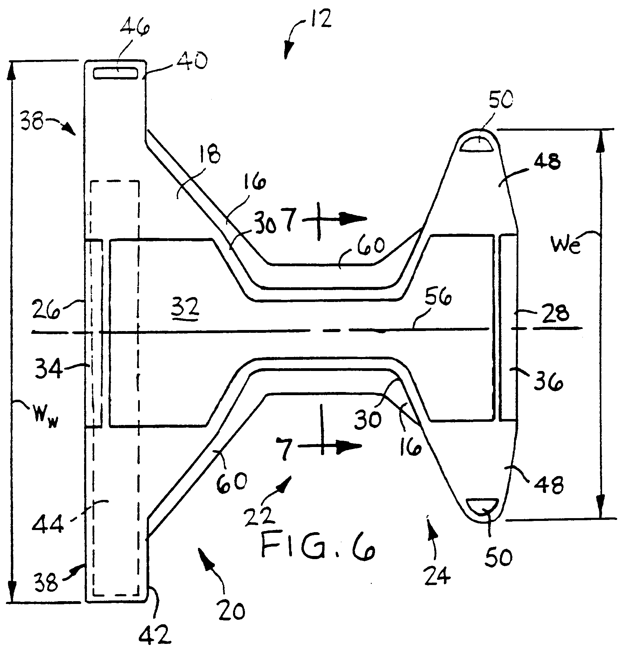

Referring to Fig. 6, diaper 12 (without a topsheet) comprises backsheet 18 having a back section 20, crotch section 22, and front section 24. Backsheet 18 further comprises back edge 26, front edge 28, and a pair of opposed lateral sides 30. Disposed on backsheet 18 are absorbent medium 32, supplemental back waist elastic 34, and supplemental front waist elastic 36. Since crotch section 22 has a narrower dimension than either front section 24 or back section 20, absorbent medium 32 in Fig. 6 has a generally hourglass shape. However, absorbent medium 32 may also have other shapes such as a rectangular shape, or a more exaggerated hourglass shape in which the ends of the absorbent extend nearer their respective lateral sides 30 of backsheet 18. Both supplemental waist elastics 34, 36 have the same maximum length, or vertical dimension as viewed in Fig. 6, as absorbent medium 32. Since diaper 12 is generally thicker in the area of absorbent medium 32, waist elastics 34, 36 assist in maintaining the ends of absorbent medium 32 nearer to the wearer's body. Supplemental waist elastics 34, 36 also may be lesser or greater in length as described above. Their lengths are generally dependent upon the type of materials of which backsheet 18 is made, the thickness and flexibility of absorbent medium 32, and the overall design of diaper 12.

A waistband 38 having opposite end portions 40, 42 can be integral with back section 20 or may be a separate structural member attached to back section 20. As illustrated in Fig. 6, waistband 38 is integral with back section 20. Since waistband 38 is to be secured about waistline 10, it will generally have a length W_{w}, as illustrated in Fig. 6, of at least about 40 centimeters. Waistband 38 includes an attachment surface 44 on its side opposite that of absorbent medium 32, and attachment panel 46 on the same side as absorbent medium 32 for attaching or securing waistband 38 about the wearer. As illustrated in dotted lines, attachment surface 44 can extend a majority of the length of waistband 38, or may just be a smaller surface area at end portion 42. Attachment surface 44 and attachment panel 46 can be a hook-and-loop fastener system in which, for example, attachment surface 44 would be a loop material and attachment panel 46 a hook material. Attachment surface 44 and attachment panel 46 can also be a cohesive material that sticks only to itself, while being non-sticky to other surfaces. Attachment surface 44 and attachment panel 46 also can be an adhesive system, or any suitable mechanical fastening system, such as a buckle, snap fasteners, and the like.
All of waistband 38 can be stretchable or, if preferred, only portions thereof, such as end portions 40, 42. Generally, if any part of waistband 38 is stretchable, that stretchable part would be a separate structural element integrated with waistband 38 or back section 20.

Front section 24 includes opposite ear portions 48, each of which includes a fastening device 50. Front section 24, like waistband 38, can also be stretchable; either all of front section 24 can be stretchable, or only portions thereof, such as ear portions 48. Front section 24 has a length, Wₑ, that is sufficiently long so that when front section 24 is brought up to the front waist portion of the wearer, ear portions 48 extend around waistline 10 to back portion 14 (Fig. 4) of the wearer to permit fastening devices 50 to fasten to attachment surface 44. If a hook-and-loop system is used on waistband 38, then fastening devices 50 would be a hook material. If a cohesive system is used with waistband 38, then fastening devices 50 would be a cohesive material.

Alternate arrangements include attachment surface 44 having one portion that is a cohesive system with attachment panel 46. Another portion of attachment surface 44 could be a loop material compatible with fastening devices 50 that would be a hook material. Or, the one portion of attachment surface 44 could be a loop material and attachment panel 46 a hook material, while the other portion of attachment surface 44 and fastening devices 50 could be a cohesive system.

In order for ear portions 48 to pass around to the back portion 14 (Fig. 4), front section 24 should have a length, Wₑ, of at least about 20 centimeters.

With reference to Fig. 4, wearer centerline 52 is illustrated in dashed lines and divides torso 2 in half, such that the portion of torso 2 to the right of centerline 52 and around the wearer to a similar point on the other side is back portion 14 of the wearer, and that portion to the left of centerline 52 and around the wearer to a similar point on the other side is front portion 54 of the wearer. As earlier described, front section 24 has a length Wₑ that permits fastening devices 50 to be passed beyond centerline 52 and attached to surface 44 at back portion 14 of the wearer. One purpose for having fastening devices 50 attached at back portion 14 of the wearer is that it moves fastening devices 50 out of the direct vision of the baby. By moving devices 50 out of the direct visual range of the baby, the baby will be less tempted to manipulate or play with fastening devices 50 or ear portions 48. This also provides a better fit because it removes the fastening devices 50 from the high stress area at the top front of the thighs where the upper legs bend near the waist. Since the fastening devices 50 are placed outside this high stress area, the tendency of front edge 28 of front section 24 to roll open or fold is minimized, if not eliminated.

Diaper 12 further comprises a stretchable leg cuff 16 (Fig. 6) at each lateral side 30. Each leg cuff 16 can vary in length, relative to longitudinal centerline 56 of diaper 12, and in shape. As illustrated in Fig. 6, leg cuffs 16 are positioned at crotch section 22 and back section 20. Leg cuffs 16 are made of a stretchable material such as a stretch-bonded laminate or an elastomeric film.

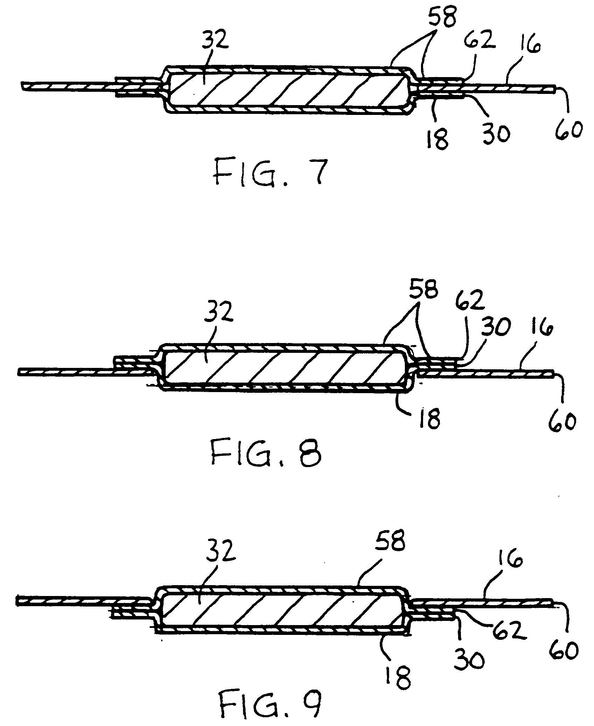

Fig. 7 is a cross-section through crotch section 22 of diaper 12 and further includes topsheet 58 on top of absorbent medium 32. Topsheet 58 may or may not have the same size and shape as backsheet 18. If topsheet 58 is utilized with diaper 12, it is preferred that it be of sufficient shape and size to permit the various attachments of leg cuffs 16 as illustrated in Figs. 7-9. Thus, as illustrated in Fig. 6, topsheet 58 would be coextensive with backsheet 18 where leg cuffs 16 reside. In Fig. 7, leg cuffs 16 are positioned between backsheet 18 and topsheet 58, such that cuff outer edge or side 60 extends laterally, outwardly beyond lateral side 30 of backsheet 18 and lateral side 62 of topsheet 58. Leg cuffs 16 can be attached to topsheet 58, or to backsheet 18, or to both topsheet 58 and backsheet 18. The attachment can be accomplished with heat sealing, adhesives, ultrasonic bonding, or any other suitable means. Leg cuffs 16 are attached so that they do not gather backsheet 18 and/or topsheet 58, relatively speaking, as do conventional diaper leg elastics that are first stretched and then attached to gather their backsheet and/or topsheet. As illustrated in Fig. 6, the portion of cuff outer side 60 at crotch section 22 is generally parallel with diaper longitudinal centerline 56. Each leg cuff 16 will easily stretch to accommodate larger babies in a specific size and weight range.

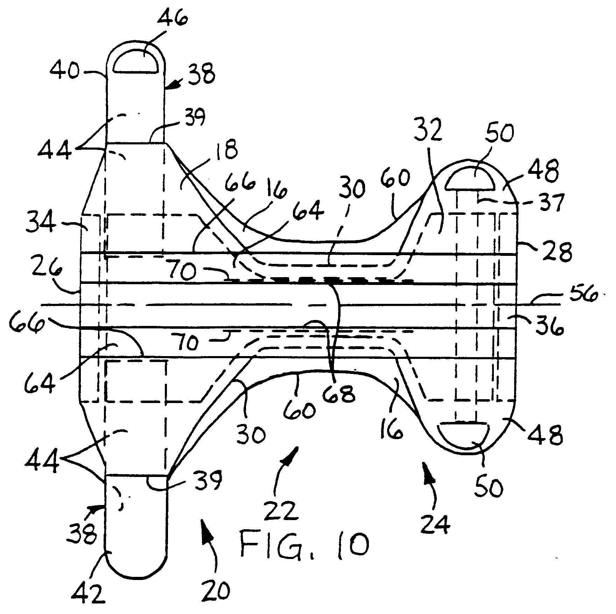

Figs. 8 and 9 show other placements of leg cuffs 16 with backsheet 18 and topsheet 58. In Fig. 9, topsheet 58 is between backsheet 18 and leg cuffs 16, and in Fig. 8, backsheet 18 is between topsheet 58 and leg cuffs 16. In all cases, cuff outer sides 60 extend laterally outwardly beyond topsheet lateral side 62 and backsheet lateral side 30.

The present invention also contemplates topsheet 58 being of smaller dimension than backsheet 18 in the areas where leg cuffs 16 reside. In this case, leg cuffs 16 would be attached to backsheet 18. Conversely, if backsheet 18 is of smaller dimension than topsheet 58, then leg cuffs 16 would be attached to topsheet 58.

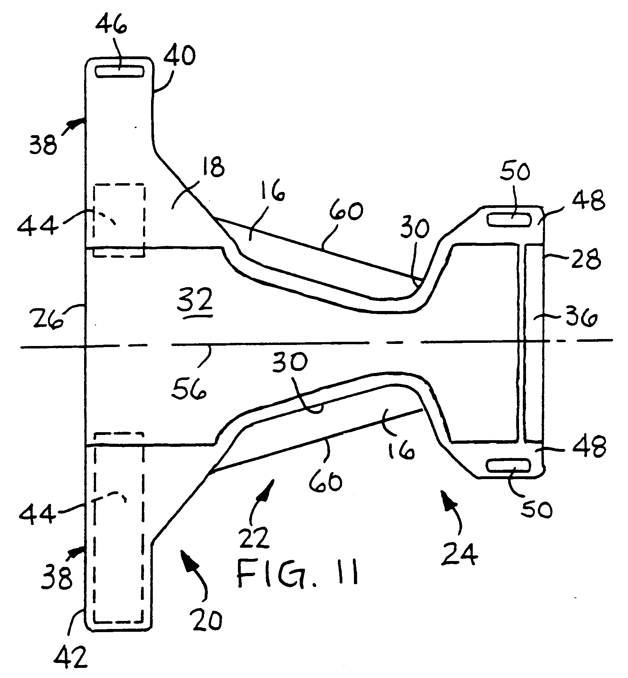

Referring to Fig. 10, there is illustrated another modification in which supplemental back waist elastic 34 is positioned longitudinally outwardly relative to waistband 38. This will improve the fit of the diaper since some mothers or caregivers have a general tendency to attach waistband 38 at the baby's hipline, rather than at the waistline 10. In the embodiment illustrated in Fig. 10, supplemental back waist elastic 34 will tend to resist diaper droop if waistband 38 is initially placed at the hipline.

Fig. 10 illustrates cuff outer sides 60 as being curvilinear. Depending upon several variables, such as baby size and material characteristics, a curvilinear design may be desired. A pair of inner containment flaps 64 are attached to topsheet 58 along their flap edges 66. Distal or free edges 68 are inboard of flap edges 66 and have elastic members 70 attached thereto in a stretched condition to cause distal edges 68 to elevate above topsheet 58. A more detailed description of this construction is contained in U.S. Pat. No. 4,704,116 to Enloe, the contents of which are incorporated by reference herein.

The modification of Fig. 10 further illustrates other features of the present invention. There are occasions when waistband 38 may not be desired to be used, such as when the child is already lying down, ill, asleep, or the like. Foldlines 39 permit end portions 40,42 to be folded inwardly. The diaper is then positioned underneath the child without fastening waistband 38 around the waist, and front section 24 is attached at back portion 14 as earlier described.

Fig. 10 also illustrates alternate front waist elastic 37 extending between fastening devices 50 to provide stretchability to front section 24. As with supplemental front and back waist elastics 34,36, alternate front waist elastic 37 is attached while in a stretched condition. Preferably, alternate front waist elastic 37 is positioned between backsheet 18 and absorbent medium 32.

Referring to Fig. 11, in another modification each leg cuff 16 has its respective cuff outer side 60 angled relative to longitudinal centerline 56 such that it diverges in a direction from front section 24 to back section 20. Absorbent medium 32 may also fan outwardly toward end portions 40, 42. This divergence of cuff outer side 60 and absorbent medium 32 increases coverage of the buttocks. The angle with which a cuff outer side 60 makes with longitudinal centerline 56 is preferably within the range between about 20° to about 70°, and will depend upon the particular size and material characteristics of the diaper.

Although Figs. 6, 10, and 11 show various modifications of the present invention, the present invention contemplates other modifications that would combine selected features. For example, diaper 12 in Fig. 6 may have the leg cuff design illustrated in Fig. 10, while the diaper illustrated in Fig. 11 may have the leg cuff design illustrated in Fig. 6. Similarly, waistband 38 may be non-stretchable, completely stretchable, or have only portions thereof that are stretchable. Front section 24 may also be stretchable, or if desired only ear portions 48 can be stretchable. In another modification, all of backsheet 18 may be stretchable. Containment flaps 64 can also be provided if desired.

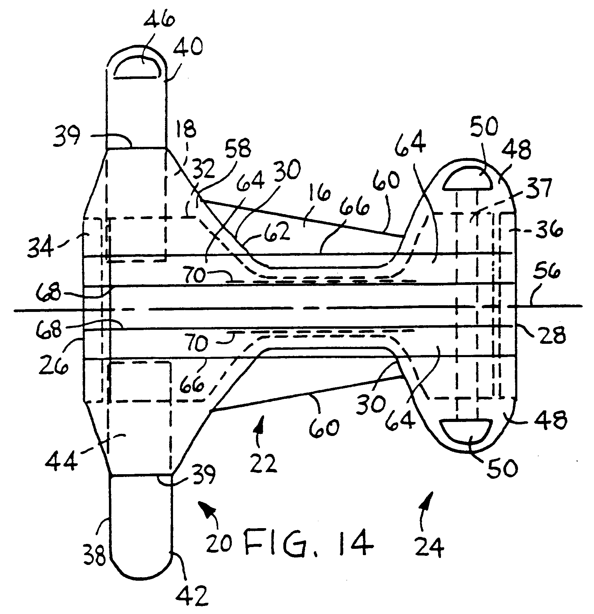

Fig. 14 illustrates a preferred embodiment of the present invention. This preferred embodiment is the diaper of Fig. 10 with the exception that leg cuffs 16 are those of the diaper in Fig. 11, i.e., cuff outer sides 60 are straight and angled relative to centerline 56. Also included, as illustrated in Fig. 10, are elasticized containment flaps 64, waist elastics 34,36, and stretchable waistband 38.

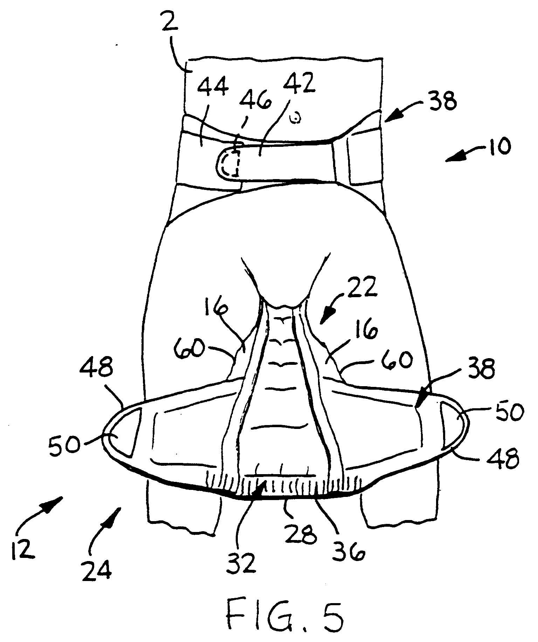

With reference to Fig. 5, diaper 12 can be fitted onto a baby that is standing by first positioning and securing waistband 38 about the waist, with the remaining portion of the diaper hanging downwardly at the back. Once waistband 38 has been comfortably secured and rotated in order to center the remaining portion of the diaper between the legs, front section 24 is brought through the legs and placed at the front of the baby with ear portions 48 then positioned and fastened at back portion 14 as illustrated in Fig. 4.

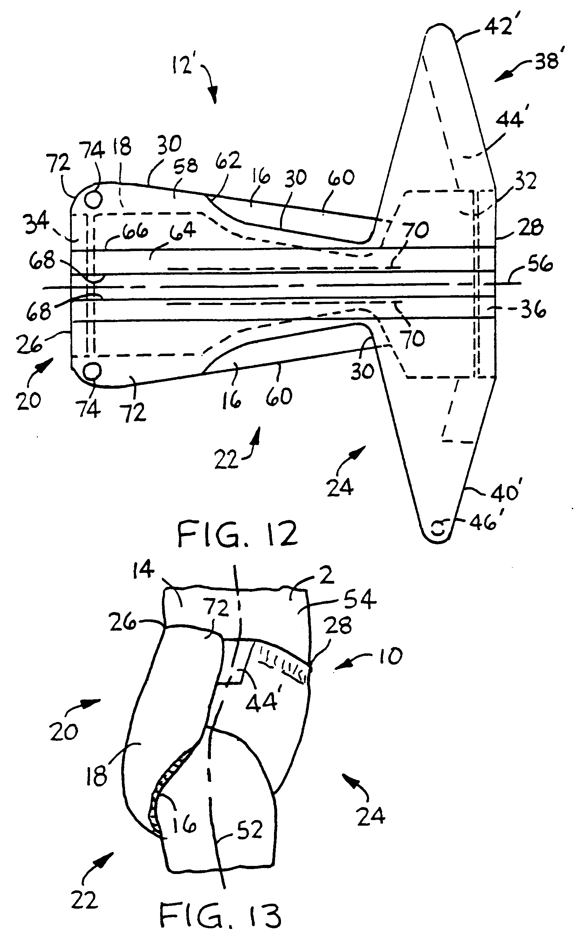

In the modification illustrated in Figs. 12 and 13, diaper 12' has waistband 38' disposed with front section 24. Waistband 38' has a length sufficient to pass around waistline 10 as described above. Back section 20 includes back ear portions 72 and fastening devices 74 for attachment to attachment surface 44' . The length of back section 20 is such that when devices 74 are attached to surface 44' , back ear portions 72 are at back portion 14 or torso 2. In placing this modification onto the baby, when waistband 38' is attached about the waist, the remaining portion of diaper 12 hangs downwardly at the front of the baby and is then passed between the legs to the back with ear portions 72 being attached to surface 44' at back portion 14. Backsheet 18 can be made of any suitable material and is preferred to be liquid impermeable. Preferably, backsheet 18 is a polyethylene film having a thickness of about 25 µm (about 1.0 mil), although thicknesses above 12.5 µm (0.50 mil) are acceptable. Backsheet 18 can be made of a material that is inherently liquid impermeable or treated to be so. Examples include meltblown or film material made of polypropylene or polyolefin copolymers such as ethylene vinyl acetate, ethylene methyl acrylate, ethylene ethyl acrylate, polyvinyl chloride, and the like. Other materials include a single spunbonded layer of the above types of material, two layers of spunbonded and meltblown materials, or three layers of material of spunbonded-meltblown-spunbonded material; each of which is suitably treated or coated to be liquid impermeable. Backsheet 18 may also be made of a material that is liquid impermeable, vapor permeable, thereby providing a breathability feature to the diaper.

Topsheet 58 and containment flaps 64 can be a liquid permeable, hydrophilic or hydrophobic material, such as a spunbonded web composed of synthetic polymer filaments; a spunlace web; a spunbond-meltblown web; a meltblown web; or a bonded carded web composed of synthetic polymer fibers. Suitable synthetic polymers include polyethylene, polypropylene, polyester, and nylon. They can also be a Kraton meltblown/polypropylene spunbond stretch-bonded laminate which has been made wettable by addition of a surfactant; and a polyurethane spunbond material. Containment flaps 64 can also be liquid impermeable and may be made of the same material as backsheet 18.

Waist elastics 34, 36; elastic members 70; and those portions of waistband 38 and front section 24 that are stretchable, can be made of any suitable elastic material, such as natural rubber, synthetic rubber, or thermoplastic elastomeric polymers, and can be panels,or single or multiple threads or filaments or ribbons thereof. These materials may also be heat-shrinkable or heat-elasticizable.

Leg cuffs 16 may also be made of any suitable material having elastic or stretchable properties. Some examples of such materials, in addition to those enumerated above, are a stretch-bonded laminate comprising two gatherable layers of about 13.5 g/m² (about 0.4 ounces per square yard) polypropylene spunbond having therebetween a layer of meltblown elastic material such as Kraton or a polyurethane-based polymer. The layer of the elastomeric is stretched, the two layers of polypropylene then are joined to the elastomeric layer, and upon relaxing the layers, the two polypropylene layers gather.

Absorbent medium 32 can be made of any suitable absorbent materials such as cellulosic fibers, synthetic fibers, absorbent gelling materials in the form of particles, fibers, layers, and the like, and various mixtures or blends thereof. Suitable absorbent gelling materials can be inorganic materials such as silica gels or organic compounds such as cross-linked polymers. Examples include polyacrylamides, polyvinyl alcohol, polyacrylates, acrylonitrile grafted starch, acrylic acid grafted starch, modified carboxy methyl cellulose, and the like. Absorbent medium 32 can also include a tissue wrap to maintain the integrity of absorbent medium 32.

## Claims

1. A disposable absorbent article (12, 12'), comprising:
a front section (24), a back section (26), a crotch section (22), and a pair of opposed lateral sides (30),
an absorbent medium (32) being disposed on said crotch section (22), a stretchable leg cuff (16) being joined to each said lateral side (30) so as to leave each said lateral side (30) relatively non-gathered, and extending laterally, outwardly beyond a respective said lateral side (30), and
an encircling waist-securing member (38, 38') disposed with one of said front section (24) and said back section (26).

2. The article of claim 1 wherein said waist-securing member (38,) is disposed on said back section (20).

3. The article of claim 1 wherein said waist-securing member (38') is disposed on said front section (24).

4. The article of any one of the preceding claims wherein said waist-securing member (38, 38') includes opposite end portions (40, 42, 40', 42') having refastening means (44, 46, 44', 46') for refastening said end portions together.

5. The article of claim 4 wherein said refastening means (44, 46, 44', 46') includes loop members on one of said end portions and hook members on the other of said end portions.

6. The article of claim 4 wherein said refastening means (44, 46, 44', 46') is a cohesive material.

7. The article of claim 4 wherein said refastening means (44, 46, 44', 46') is a mechanical fastener.

8. The article of any one of the preceding claims further comprising a waist elastic member (34, 36) being adjacent said waist-securing member (38, 38').

9. The article of any one of the preceding claims wherein at least a portion of said waist-securing member (38, 38') is stretchable.

10. The article of any one of the preceding claims wherein said waist-securing member (38, 38') has an extended length of at least about 40 centimeters.

11. The article of claim 2 wherein said front section (24) includes opposed ear portions (48), each said ear portion (48) having a first engaging means thereon (50), and wherein said back section (20) includes a second engaging means (44) thereon, a first engaging means (50) and a second engaging means (44) being releasably engageable together.

12. The article of claim 11 wherein said first (50) and said second (44) engaging means are a plurality of hook members and a plurality of loop members.

13. The article of claim 11 wherein said first and said second engaging means are a cohesive material.

14. The article of any one of claims 11 to 13 wherein said second engaging means (44) is disposed on said back section (20) such that said second engaging means is adapted to be at a back portion (14) of a wearer, and
wherein said ear portions (48) are of a length adapted for passing around to the back portion (14) of the wearer, whereby said first engaging means (50) is releasably engageable to said second engaging means (44) at the back portion (14) of the wearer.

15. The article of any one of claims 11 to 14 wherein at least a portion of said front section (24) is stretchable.

16. The article of any one of claims 11 to 15 wherein said ear portions (48) are stretchable.

17. The article of claim 3 wherein said waist-securing member (38') includes refastenable opposite end portions (40', 42'), at least one of said end portions including a first engaging means (46') thereon, and wherein said back section (20) includes a second engaging means (74) being releasably engageable with said first engaging means (46').

18. The article of claim 17 wherein said first engaging means (46') is disposed on said at least one end portion (40', 42') such that said first engaging means (46') is adapted to be at a back portion (14) of a wearer when being releasably engaged with said second engaging means (74).

19. The article of claim 17 or 18 wherein at least a portion of said back section (20) is stretchable.

20. A disposable absorbent article (12) especially according to claim 1, comprising:
a front section (24), a back section (20), a crotch section (22), and a pair of opposed lateral sides (30),
an absorbent medium (32) being disposed on said crotch section (22),
a stretchable leg cuff (16) being joined to each said lateral side (30),
said front section (24) including an extended length portion (48) that is extendable around to a back portion (14) of a wearer,
a first engaging means (50) on said extended length portion of said front section (24), and
a second engaging means (44) on said back section (20) and being releasably engageable with said first engaging means (50) at the back portion (14) of the wearer.

21. The article of claim 20 wherein said engaging means (50, 44) are hook and loop members.

22. The article of claim 20 wherein said engaging means (50, 44) are a cohesive material.

23. The article of claim 20 wherein said engaging means (50, 44) is a mechanical fastener.

24. The article of any one of claims 20 to 23 wherein at least a portion of said front section (24) is stretchable.

25. The article of any one of claims 20 to 24 wherein each said leg cuff (16) extends laterally outwardly beyond a respective said lateral side (30).

26. A disposable absorbent article (12), comprising:
a front section (24), a back section (20), a crotch section (22), and a pair of lateral sides (30),
an absorbent medium (32) being disposed on said crotch section (22), and
a pair of stretchable leg cuffs (16) being disposed on respective ones of said lateral sides (30) so as to leave said lateral sides relatively non-gathered, each said stretchable leg cuff (10) having a stretchable outer edge portion (60) extending laterally, outwardly beyond a respective said lateral side (30),
said front section (24) and said back section (20) being releasably refastenable together for fastening said article on a wearer.

27. The article of claim 26 wherein said front section (24) comprises a pair of ear portions (48) having first fastening means (50) thereon and said back section (20) comprises a second fastening means (44) for being releasably fastenable with said first fastening means (50) on each said ear portion (48), said first (50) and said second (44) fastening means being releasably refastenable at a back portion (14) of the wearer.

28. The article of claim 27 wherein said first fastening means are a plurality of hook members and said second fastening means are a plurality of loop members.

29. The article of any one of claims 26 to 28 further comprising an encircling waist member (38) being disposed on said back section (20) and having thereon securing means (44, 46) adapted for securing said waist member about a waist of a wearer.

30. The article of claim 29 wherein said securing means (44, 46) comprises a plurality of hook members and a plurality of loop members.

31. The article of claim 29 wherein said securing means (44, 46) comprises a cohesive material.

32. The article of any one of claims 29 to 31 wherein said waist member (38) has at least a portion thereof that is stretchable.

33. The article of any one of claims 26 to 32 wherein said ear portions (48) are stretchable.

34. The article of any one of claims 26 to 33 wherein each said leg cuff (16) is a stretch bonded laminate.

35. The article of any one of the preceding claims wherein said leg cuffs (16) are a film of elastomeric material.

36. The article of any one of claims 1 to 34 wherein said leg cuffs (16) are a stretch-bonded laminate.

37. The article of any one of the preceding claims wherein each said leg cuff (16) has a side edge (60) spaced laterally outwardly from a respective said lateral side (30), a portion of said side edge (60) at said crotch section (22) being generally parallel to a longitudinal centerline (56) of said crotch section (22).

38. The article of any one of claims 1 to 36 wherein each said leg cuff (16) has a side edge (60) spaced laterally outwardly from a respective said lateral side (30), a portion of said side edge (60) at said crotch section being angularly disposed to a longitudinal centerline (56) of said crotch section (22).

39. The article of claim 38 wherein said side edge portion (60) is at an angle with the longitudinal centerline between 20° to 70°.

40. The article of any one of claims 1 to 36 wherein each said leg cuff (16) has a side edge (60) spaced laterally outwardly from a respective said lateral side (30), a portion of said side edge at said crotch section being curvilinear.

41. The article of any one of the preceding claims further comprising a backsheet (18) and a topsheet (58), said absorbent medium (32) being between said topsheet (58) and said backsheet (18), and
wherein each said leg cuff (16) is disposed between said topsheet (58) and said backsheet (18).

42. The article of any one of claims 1 to 40 further comprising a backsheet (18) and a topsheet (58), said absorbent medium (32) being between said topsheet (58) and said backsheet (18), and
wherein each said leg cuff (16) is disposed on a side of said topsheet (58) opposite from said absorbent medium (32).

43. The article of any one of claims 1 to 40 further comprising a backsheet (18) and a topsheet (58), said absorbent medium (32) being between said topsheet (58) and said backsheet (18), and
wherein each said leg cuff (16) is disposed on a side of said backsheet (18) opposite from said absorbent medium (32).
